# EUROPÄISCHE PATENTSCHRIFT

(11) **EP 2 818 147 B1**
(45) Veröffentlichungstag und Bekanntmachung des Hinweises auf die Patenterteilung: **02.10.2019**
(21) Anmeldenummer: 14171962.5
(22) Anmeldetag: 11.06.2014
(51) Int. Cl.: A61F 13/15

(54) **Verfahren und Anlage zur Herstellung von Hygieneartikeln**
Method and device for manufacturing hygiene articles
Procédé et système de fabrication d'articles hygiéniques

(30) Priorität: 24.06.2013 DE 102013211945
(43) Veröffentlichungstag der Anmeldung: 31.12.2014
(73) Patentinhaber: Winkler + Dünnebier GmbH, 56564 Neuwied (DE)
(72) Erfinder: Hartmann, Werner, 56271 Kleinmaischeid (DE); Siebert, Jakob, 56564 Neuwied (DE)
(74) Vertreter: Tergau & Walkenhorst Patentanwälte PartGmbB

(56) Entgegenhaltungen:
- EP-A1- 0 958 801
- EP-A1- 2 508 156
- DE-A1-102004 043 289
- DE-C1- 10 123 099

## Beschreibung

Die Erfindung bezieht sich auf ein Verfahren zur Herstellung von Hygieneartikeln mit jeweils einem ober- und unterseitig jeweils mit einer oder mehreren Lagen von Materialbahnen versehenen Absorberkern, bei dem ein aus einem zur Bildung der Absorberkerne vorgesehenen Gemisch gebildeter portionierter Materialstrom zunächst oberseitig und dann unterseitig jeweils mit einer beleimten Materialbahn versehen wird. Sie betrifft weiter eine Anlage zur Durchführung des Verfahrens.

Hygieneprodukte wie beispielsweise Damenbinden oder Windeln sind üblicherweise zur Aufnahme und zumindest vorübergehenden Speicherung von Körperflüssigkeiten ausgelegt. Dazu umfassen derartige Hygieneprodukte einen Absorberkörper oder Absorberkern, der als wesentliche Komponenten insbesondere saugfähiges Material, üblicherweise in Form von Fasern, enthält. Im Hinblick auf die gewünschten Eigenschaften hinsichtlich der Aufnahmefähigkeit von Flüssigkeiten kommen dabei zur Bildung des Absorberkerns üblicherweise geeignet gewählte Materialmischungen zum Einsatz, die zusätzlich zu Zellstofffasern oder dergleichen so genannte superabsorbierende Materialien enthalten. Der Absorberkern wird anschließend zwischen geeigneten Material- oder Gewebelagen angeordnet und von ihnen eingeschlossen. Diese Materiallagen bilden somit die äußere Haut oder Hülle des jeweiligen Hygieneprodukts.

Das superabsorbierende Material kann dabei als Pulver oder auch, wie beispielsweise aus der DE 10 2006 000 779 bekannt, als Faserkomponente vorliegen und wird bei der Herstellung des Absorberkerns zunächst den Zellstofffasern beigemischt. Die dabei entstehende Materialmischung dient als Basismaterial zur Herstellung des Absorberkerns und wird in Form eines Materialstroms einer Weiterverarbeitung zugeführt. Dabei wird die Materialmischung zunächst geeignet portioniert und einer Formgebung unterzogen, so dass der eigentliche Absorberkörper entsteht. Dieser wird in einem anschließenden Verarbeitungsschritt beidseitig mit den geeignet gewählten Material- oder Gewebelagen versehen, und das dadurch entstehende fertige Hygieneprodukt wird anschließend geeignet verkaufsfähig verpackt.

Die Herstellungsprozesse werden dabei üblicherweise in geeignet automatisierten, für hohe Stückzahlen ausgelegten Anlagen durchgeführt. Im Hinblick auf die hohen Stückzahlen und die gemessen am Endprodukt vergleichsweise hohen Anteile an Materialkosten ist dabei eine effiziente und ressourcenschonende Prozessführung besonders bedeutsam. Insbesondere ist dabei im Hinblick auf die angestrebten hohen Durchsatzraten wünschenswert, den Prozessablauf weitgehend frei von Verunreinigungen, Materialresten und dergleichen zu halten, so dass dadurch bedingte Beeinträchtigungen oder Störungen vermieden werden können.

Der Erfindung liegt daher die Aufgabe zugrunde, ein Verfahren zur Herstellung von Hygieneprodukten der oben genannten Art anzugeben, das eine derartige Prozessführung mit einfachen Mitteln besonders begünstigt. Weiter soll eine zur Durchführung des Verfahrens besonders geeignete Anlage angegeben werden.

Bezüglich des Verfahrens wird diese Aufgabe erfindungsgemäß gelöst, indem der mit der oberseitigen Materialbahn versehene Materialstrom mittels eines oberseitig angeordneten Transportmittels zu einer Zuführstation für die unterseitige Materialbahn transportiert und während dieses Transports nach unten hin von einem mitlaufenden Siebband abgestützt wird.

Die Erfindung geht dabei von der Überlegung aus, dass eine besonders präzise und störungsfreie Betriebsweise bei der Herstellung der Hygieneprodukte gerade im Hinblick auf die hohen Stückzahlen und Durchsatzraten erreichbar ist, indem an geeigneten Stellen im Prozessablauf Materialreste, Verunreinigungen und dergleichen zuverlässig entfernt werden. In diesem Zusammenhang sollte unter anderem berücksichtigt werden, dass nach der Portionierung und Formgebung der zur Bildung der Absorberkerne vorgesehenen Gemischchargen noch überschüssige Partikel, Fragmente oder dergleichen vorliegen könnten, die die nachfolgenden Prozesschritte in unerwünschter Weise beeinträchtigen könnten. Insbesondere kann es nach der Vermischung der Zellstofffasern mit den Absorberpartikeln vorkommen, dass noch einzelne Partikel oder Fragmente vorliegen, die nicht sauber in den eigentlichen Absorberkern eingebunden sind und sich in späteren Prozessschritten aus dem Verbund herauslösen und zur Störquelle werden könnten. Insbesondere könnten derartige Partikel oder Fragment im Kantenbereich der den Absorberkern später umgebenden Materiallagen vorliegen und/oder aus der durch diese gebildeten Umhüllung austreten, was insbesondere bei den Endverbrauchern als unangenehm und störend wahrgenommen werden könnte.

Um dem entgegenzuwirken, sollte nach der Portionierung und Formgebung der Absorberkerne eine zuverlässige Entfernung nicht fest eingebundener Partikel, Fragmente oder Materialreste vorgesehen sein. Dies ist besonders zuverlässig und mit einfachen Mitteln möglich, indem die Absorberkerne nach ihrer Portionierung und Formgebung, aber vor der endgültigen Einbettung in die sie umgebende Hülle vorübergehend in der Art einer "hängenden" Aufhängung und ggf. sogar unter zusätzlich aufgebrachter mechanischer Beanspruchung (bevorzugt beispielsweise durch Rütteln oder dergleichen) transportiert werden, so dass unter gezielter Nutzung der Schwerkraft die unerwünschten losen Partikel angeschieden oder entfernt werden können. Um dies zu ermöglichen, ist in einem ersten Schritt bei der Einbindung der Absorberkerne in die sie später umgebenden Materialbahnen die Aufbringung der oberen Materialbahn vorgesehen. Dazu wird zunächst die obere, beleimte Materialbahn zugeführt, an der die Absorberkerne (vorzugsweise durch Andrücken) fixiert werden.

Anschließend werden die nun an der oberseitigen Materialbahn befestigten Absorberkerne in der Art einer hängenden Anordnung mit einem von oben angreifenden Transportsystem weitertransportiert. Dadurch ist ermöglicht, dass in diesem Transportschritt das noch lose Material oder die losen Partikel bedingt durch die Schwerkraft nach unten hin abfallen und anschließend entsorgt werden können. Nach diesem Transportschritt, in dem die unerwünschten losen Fragmente oder Partikel "abgeschüttelt" werden, kann die Zuführung der unteren Materialbahn erfolgen, die sodann gemeinsam mit der oberseitigen Materialbahn die Umhüllung des jeweiligen Absorberkerns bildet. Um dabei auch während dieses Transportschritts eine besonders zuverlässige Prozessführung zu ermöglichen, bei der der Produktstrom durchgängig im Eingriff geführt wird, ist eine Abstützung nach unten über ein Siebband vorgesehen, durch das die losen Partikel nach unten hin hindurchfallen können.

Unter "Siebband" im hier verwendeten Sinne ist dabei ein Transportband zu verstehen, dass im Hinblick auf den bestimmungsgemäßen Gebrauch geeignet ausgestaltet ist, dass die zu entfernenden Partikel geeignet hindurchfallen können. Dazu umfasst das Siebband ein Laufband, dessen Bandfläche mit offenen Poren oder Löchern versehen ist, die das Hindurchfallen der Partikel zulassen. In der Art eines konventionellen Siebbandes kann das Laufband daher in der Art einer Siebstruktur auf metallischer oder auch Kunststoffbasis ausgeführt sein, bei der die Löcher von Streben oder dergleichen gebildet oder begrenzt sind. Alternativ kann das Siebband aber auch als offenporiges oder mit vergleichsweise großen Öffnungen versehenes Gummiband, als Zahnriemen mit geeignet groß gewählten Öffnungen oder als andersartig bestimmungsgemäß geeignet ausgeführtes Band ausgestaltet sein.

Besonders vorteilhaft für eine zuverlässige Prozessführung ist hierbei die Abstützfunktion des Siebbandes, da hierdurch auch bei der Beförderung eines nicht vollflächig mit der oberen Materialbahn verleimten Materialstroms ein zuverlässiger Transport möglich ist, ohne dass Teile oder Stücke des Materialstroms nach unten durchhängen könnten.

In besonders vorteilhafter Ausgestaltung wird als Transportmittel für den Transport des mit der oberseitigen Materialbahn versehenen Materialstroms ein vakuumbeaufschlagtes Saugband verwendet. Dieses erlaubt einen zuverlässigen Zugriff auf die oberseitige Materialbahn ausschließlich von oben her, so dass ein Abstreifen oder Herauslösen der losen Partikel nach unten hin nahezu ungehindert möglich ist.

Zur Bildung der Absorberkerne wird besonders bevorzugt ein Gemisch aus Zellstofffasern mit einem superabsorbierenden Material verwendet.

Bezüglich der Anlage zur Herstellung von Hygieneartikeln wird die genannte Aufgabe gelöst mit einer ersten Zuführstation zur Zuführung einer oberseitigen Materialbahn zu einem zur Bildung von Absorberkernen vorgesehenen portionierten Materialstrom, mit einer dieser materialstromseitig nachgeschalteten zweiten Zuführstation für die unterseitige Materialbahn, und mit einem Transportsystem zur Überführung des mit der oberseitigen Materialbahn versehenen Materialstroms von der ersten zur zweiten Zuführstation, das als die oberseitige Materialbahn von oben tragendes System ausgeführt ist.

In besonders vorteilhafter Ausgestaltung umfasst das Transportsystem dabei ein vakuumbeaufschlagtes Saugband. Dieses ermöglicht einen zuverlässigen Zugriff auf die oberseitige Materialbahn, ohne dabei das schwerkraftbedingte Herabfallen der losen Partikel aus dem Absorberkern nach unten zu behindern.

Zur Abstützung des mit der oberseitigen Materialbahn versehenen Materialstroms von unten ist dem Transportsystem in besonders vorteilhafter Ausgestaltung ein mitlaufendes Siebband zugeordnet. Dieses weist auf seiner Lauffläche in weiterer vorteilhafter Ausgestaltung und insbesondere im Hinblick auf die besonders bevorzugt abzustreifenden Partikelgrößen eine Siebfläche mit einer Maschenweite von mindestens 1 mm, vorzugsweise von mindestens 2mm und/oder höchstens 5mm, besonders bevorzugt von 3 mm oder 4 mm, auf. Eine größere oder kleinere Maschenweite ist ebenso denkbar, falls die abzustreifenden Partikel eine entsprechende Partikelgröße aufweisen. Insbesondere kann in einer Variante der Erfindung auch eine gezielte Auslegung zum Abstreifen besonders kleiner Partikelgrößen vorgesehen sein, wobei ein Unterstützungsband mit entsprechend klein dimensionierten Öffnungen vorgesehen sein kann.

Vorteilhafterweise ist die Anlage, insbesondere im Bereich ihres Transportsystems, noch mit weiteren Systemen, beispielsweise mit einer Rüttlereinheit oder dergleichen, versehen, die das Herauslösen und Herabfallen loser Partikel oder Fragmente aus dem Absorberkern während des Transports zur zweiten Zuführstation noch weiter begünstigt. In alternativer oder zusätzlicher vorteilhafter Ausgestaltung ist dem Transportsystem zudem noch eine Absaugeinrichtung zugeordnet, mit der lose Partikel oder Fragmente aus den Absorberkernen abgesaugt werden können. Damit kann durch die Absaugung die schwerkraftbedingte Entfernung der losen Partikel oder Fragmente aus den an der oberseitigen Materialbahn hängend transportierten Absorberkernen noch zusätzlich unterstützt werden.

Die mit der Erfindung erzielten Vorteile bestehen insbesondere darin, dass durch den Transportschritt, mit dem die Absorberkerne - lediglich an der oberseitigen beleimten Materialbahn befestigt und an dieser hängend - zur für die unterseitige Materialbahn vorgesehenen zweiten Zuführstation überführt werden, auf apparativ besonders einfache und dennoch zuverlässige Weise unter gezielter Nutzung der Schwerkraft lose Partikel oder Fragmente vom Absorberkern entfernt werden können. Damit können spätere Beeinträchtigungen oder Störeinflüsse durch solche Partikel ode Fragmente, beispielsweise ein späteres Austreten aus der durch die Materialbahnen gebildeten Umhüllung des Absorberkerns, zuverlässig vermieden werden. Im Übrigen ist die Erfindung vorstehend und nachfolgend am Beispiel einer Anwendung im Rahmen der Herstellung von Hygieneartikeln beschrieben. Selbstverständlich kann das erfinderische Konzept, ggf. unter angepasster Wahl der Lochgrößen im Unterstützungsband, aber auch bei der Herstellung oder im Verarbeitungsprozess anderer vorkomprimierter Granulatartikel oder dergleichen vorteilhaft zum Einsatz kommen. Zudem ist durch die Abstützfunktion des Siebbandes, die selbst bei der Beförderung eines nicht vollflächig mit der oberen Materialbahn verleimten Materialstroms zuverlässig verhindert, dass Teile oder Stücke des Materialstroms nach unten durchhängen könnten, auf besonders kostengünstige Weise eine hohe Prozesssicherheit erreichbar.

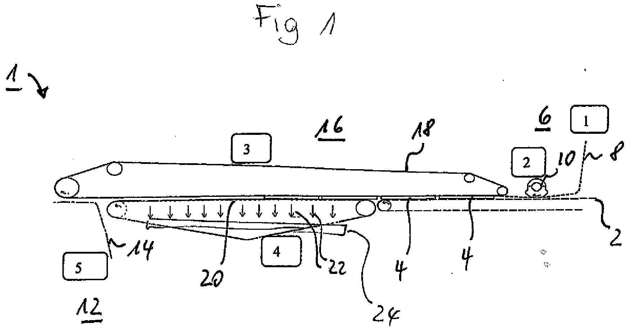

Ein Ausführungsbeispiel der Erfindung wird anhand einer Zeichnung näher erläutert. Darin zeigt die Figur schematisch eine Anlage zur Herstellung von Hygieneartikeln.

In der Figur ist die Aufbringungseinheit 1 für Gewebelagen einer zur Herstellung von Hygieneartikeln vorgesehenen Anlage gezeigt. In dieser Anlage wird in einer nicht dargestellten Mischvorrichtung ein erster Materialstrom aus Zellstofffasern mit einem zweiten Materialstrom aus einem superabsorbierenden Material vermischt. In einem vorgelagerten Behandlungsschritt wird zur Bereitstellung des ersten Materialstroms eine Materialbahn von einer Materialtrommel abgewickelt und in einer Mühle zerkleinert, so dass das Material als faseriges, rieselfähiges Grundmaterial vorliegt. Der zweite Materialstrom aus superabsorbierendem Material wird hingegen aus einem Vorlagebehälter entnommen und der Mischvorrichtung zugeführt.

Aus der Mischvorrichtung wird das Gemisch entnommen und als zur Bildung der Absorberkerne vorgesehener Materialstrom einer Portionier- und Formgebungseinheit zugeführt. In dieser wird der Materialstrom portioniert, und den jeweiligen Portionen wird eine für die vorgesehene Verwendung als Absorberkern geeignete Form gegeben. Die solchermaßen geformten Absorberkerne können anschließend ggf. in einer Kompressionseinheit geeignet komprimiert werden. Anschließend sind die Absorberkerne für die Aufbringung der Gewebelagen in der nachfolgend angeordneten Aufbringungseinheit 1 vorbereitet. Im Ausführungsbeispiel sind dabei Gewebelagen vorgesehen; alternativ oder zusätzlich können als so genanntes "Top-Sheet" und/oder "Back-Sheet" aber auch ein- oder mehrlagige Materialbahnen anderer Materialien wie beispielsweise Kunststoffe vorgesehen sein.

In der Aufbringungseinheit 1 werden die als portionierter Materialstrom 2 vorliegenden Absorberkerne 4 zunächst in einer materialstromseitig gesehen ersten Zuführstation 6 mit einer beleimten oberseitigen Materialbahn 8 zusammengeführt. Die beleimte oberseitige Materialbahn 8 wird dabei mittels einer angetriebenen Nockenwalze 10 an die den Materialstrom 2 bildenden Absorberkerne 4 angedrückt, so dass die Absorberkerne 4 an der beleimten Materialbahn 8 anhaften. Von der ersten Zuführstation 6 aus wird die mit den Absorberkernen 4 versehene Materialbahn 8 an eine materialstromseitig gesehen nachgeschaltete zweite Zuführstation 12 übergeben und dort mit einer ebenfalls beleimten unterseitigen Materialbahn 14 zusammengeführt. Anschließend können die fertig hergestellten Produkte geeignet aus der Anlage ausgeschleust werden.

Die Aufbringungseinheit 1 ist gezielt für einen besonders störungsfreien Betrieb mit einfachen Mitteln ausgeführt. Dabei ist insbesondere dem Umstand Rechnung getragen, dass nach der Portionierung und Formgebung der Absorberkerne 4 noch überschüssige Partikel, Fragmente oder dergleichen vorliegen könnten, die als Verschmutzungen die nachfolgenden Prozesschritte in unerwünschter Weise beeinträchtigen könnten. Insbesondere kann es nach der Vermischung der Zellstofffasern mit den Absorberpartikeln vorkommen, dass noch einzelne Partikel oder Fragmente vorliegen, die nicht sauber in den eigentlichen Absorberkern eingebunden sind und sich in späteren Prozessschritten aus dem Verbund herauslösen und zur Störquelle werden könnten. Um dem entgegenzuwirken, ist die Aufbringungseinheit 1 gezielt für eine zuverlässige Entfernung derartiger nicht fest eingebundener Partikel, Fragmente oder Materialreste mit besonders einfachen Mitteln, nämlich insbesondere unter Nutzung der Schwerkraft, ausgeführt.

Hierzu ist in der Aufbringungseinheit 1 der Transport der bereits mit der oberseitigen Materialbahn 8 versehenen Absorberkerne 4 in hängender Anordnung zur nachfolgenden zweiten Zuführstation 12 vorgesehen, so dass lose und überschüssige Partikel problemlos nach unten hin abfallen können. Zu diesem Zweck ist das Transportsystem 16 der Aufbringungseinheit 1, mit dem die Überführung des mit der oberseitigen Materialbahn 8 versehenen Materialstroms 2 von der ersten Zuführstation 6 zur zweiten Zuführstation 12 durchgeführt wird, als die oberseitige Materialbahn 8 von oben tragendes System, nämlich als vakuumbeaufschlagtes Saugband 18, ausgeführt.

Zur Abstützung des mit der oberseitigen Materialbahn 8 versehenen Materialstroms 2 von unten während dieser Überführung umfasst das Transportsystem 16 weiterhin ein mitlaufendes Siebband 20. Dieses weist auf seiner Lauffläche eine Siebfläche mit einer Maschenweite von etwa 3 oder 4 mm auf. Durch diese Parameterwahl ist sichergestellt, dass bei zuverlässiger mechanischer Abstützung von unten dennoch ein vergleichsweise problemloses Abwerfen der losen Partikel, wie durch die Pfeile 22 angedeutet, möglich ist. Durch die geignete Wahl der Maschenweite werden somit zuverlässig lose Partikel und auch Staub abgesaugt.

Um dies noch weiter zu unterstützen, ist dem Transportsystem 16 zudem eine Absaugeinrichtung 24 zur Absaugung von losen Partikeln oder Fragmenten aus den Absorberkernen 4 zugeordnet.

### Bezugszeichenliste

- 1: Aufbringungseinheit
- 2: Materialstrom
- 4: Absorberkern
- 6: erste Zuführstation
- 8: oberseitige Materialbahn
- 10: Nockenwalze
- 12: zweite Zuführstation
- 14: unterseitige Materialbahn
- 16: Transportsystem
- 18: Saugband
- 20: Siebband
- 22: Pfeil
- 24: Absaugeinreichtung

## Patentansprüche

1. Verfahren zur Herstellung von Hygieneartikeln mit jeweils einem ober- und unterseitig jeweils mit einer oder mehreren Lagen von Materialbahnen (8, 14) versehenen Absorberkern (4), bei dem ein aus einem zur Bildung der Absorberkerne (4) vorgesehenen Gemisch gebildeter portionierter Materialstrom (2) zunächst oberseitig und dann unterseitig jeweils mit einer beleimten Materialbahn (8, 14) versehen wird, wobei der mit der oberseitigen Materialbahn (8) versehene Materialstrom (2) mittels eines oberseitig angeordneten Transportmittels zu einer Zuführstation (12) für die unterseitige Materialbahn (14) transportiert und während dieses Transports nach unten hin von einem mitlaufenden Siebband (20) abgestützt wird.

2. Verfahren nach Anspruch 1, bei dem als Transportmittel für den Transport des mit der oberseitigen Materialbahn (8) versehenen Materialstroms (2) ein vakuumbeaufschlagtes Saugband (18) verwendet wird.

3. Verfahren nach Anspruch 1 oder 2, bei dem zur Bildung der Absorberkerne (4) ein Gemisch aus Zellstofffasern mit einem superabsorbierenden Material verwendet wird.

4. Anlage (1) zur Herstellung von Hygieneartikeln, insbesondere zur Durchführung des Verfahrens nach einem der Ansprüche 1 bis 3, mit einer ersten Zuführstation (6) zur Zuführung einer oberseitigen Materialbahn (8) zu einem zur Bildung von Absorberkernen (4) vorgesehenen portionierten Materialstrom (2), mit einer dieser materialstromseitig nachgeschalteten zweiten Zuführstation (12) für die unterseitige Materialbahn (14), und mit einem Transportsystem (16) zur Überführung des mit der oberseitigen Materialbahn (8) versehenen Materialstroms (2) von der ersten zur zweiten Zuführstation (12), das als die oberseitige Materialbahn (8) von oben tragendes System ausgeführt ist.

5. Anlage (1) nach Anspruch 5, deren Transportsystem (16) ein vakuumbeaufschlagtes Saugband (18) umfasst.

6. Anlage (1) nach Anspruch 4 oder 5, deren Transportsystem (16) zur Abstützung des mit der oberseitigen Materialbahn (8) versehenen Materialstroms (2) von unten ein mitlaufendes Siebband (20) zugeordnet ist.

7. Anlage (1) nach Anspruch 6, deren mitlaufendes Siebband (20) auf seiner Lauffläche eine Siebfläche mit einer Maschenweite von mindestens 1 mm, vorzugsweise von mindestens 2mm und/oder höchstens 5mm, aufweist.

8. Anlage (1) nach einem der Ansprüche 4 bis 7, deren Transportsystem (16) eine Absaugeinrichtung (24) zur Absaugung von losen Partikeln oder Fragmenten aus den Absorberkernen zugeordnet ist.

## Claims

1. Method for producing hygiene articles, each having an absorber core (4) which is provided on the upper and lower sides thereof with one or more layers of material webs (8, 14), in which method a portioned material stream (2), formed from a mixture provided for forming the absorber cores (4), is provided in each case with a glued material web (8, 14), firstly on the upper side and then on the lower side, wherein the material stream (2) provided with the upper-side material web (8) is transported by means of a transport means, arranged on the upper side, to a feed station (12) for the lower-side material web (14) and, during this transport, is supported below by a travelling screen belt (20).

2. Method according to claim 1, wherein a suction belt (18), to which a vacuum is applied, is used as the transport means for transporting the material stream (2) provided with the upper-side material web (8).

3. Method according to either claim 1 or claim 2, wherein a mixture of pulp fibres with a superabsorbent material is used to form the absorber cores (4).

4. Plant (1) for the production of hygiene articles, in particular for carrying out the method according to any of claims 1 to 3, comprising a first feed station (6) for feeding an upper-side material web (8) to a portioned material stream (2) provided for forming absorber cores (4), comprising a second feed station (12), arranged on the material stream side so as to be downstream of this first feed station, for the lower-side material web (14), and comprising a transport system (16) for transferring the material stream (2) provided with the upper-side material web (8) from the first to the second feed station (12), which system is designed as the system that carries the top-side material web (8) from above.

5. Plant (1) according to claim 5, the transport system (16) of which comprises a suction belt (18) to which a vacuum is applied.

6. Plant (1) according to either claim 4 or claim 5, the transport system (16) of which is associated with a travelling screen belt (20) for supporting the material stream (2), provided with the upper-side material web (8), from below.

7. Plant (1) according to claim 6, the travelling screen belt (20) of which has, on its running surface, a screen surface having a mesh size of at least 1 mm, preferably of at least 2 mm and/or at most 5 mm.

8. Plant (1) according to any of claims 4 to 7, the transport system (16) of which is associated with a suction device (24) for the extraction of loose particles or fragments from the absorber cores.

## Revendications

1. Procédé de fabrication d'articles hygiéniques comportant chacun un noyau absorbant (4) sur les côtés supérieur et inférieur, lesquels côtés comportant respectivement au moins une couche de bandes de matériau (8, 14), un courant de matériau en portions (2) constitué d'un mélange prévu pour former les noyaux absorbants (4) comportant au préalable sur le côté supérieur, puis sur le côté inférieur respectivement, une bande de matériau encollée (8, 14), le courant de matériau (2) comportant la bande de matériau supérieure (8) étant transporté à l'aide d'un moyen de transport disposé sur le côté supérieur vers une station d'alimentation (12) de la bande de matériau inférieur (14), et pendant ce transport, ledit courant de matériau étant supporté vers le bas par un tamis sans fin mobile (20).

2. Procédé selon la revendication 1, dans lequel une bande aspirante (18) sollicitée par le vide est utilisée comme moyen de transport destiné au transport du courant de matériau (2) comportant la bande de matériau supérieure (8).

3. Procédé selon la revendication 1 ou 2, dans lequel un mélange de fibres de cellulose comportant un matériau superabsorbant permet de former les noyaux absorbants (4).

4. Installation (1) de fabrication d'articles hygiéniques, en particulier de mise en oeuvre du procédé selon l'une des revendications 1 à 3, comportant une première station d'alimentation (6) destinée à amener une bande de matériau supérieure (8) vers un courant de matériau (2) en portions prévu pour former des noyaux absorbants (4), une seconde station d'alimentation (12) destinée à la bande de matériau inférieure (14) montée en aval de ladite première station de matériau du côté du courant de matériau, et un système de transport (16) destiné à transférer le courant de matériau (2) comportant la bande de matériau supérieure (8) de la première à la seconde station d'alimentation (12), lequel système de transport est conçu comme un système portant la bande de matériau supérieure (8) par le haut.

5. Installation (1) selon la revendication 5, dont le système de transport (16) comprend une bande aspirante (18) sollicitée par le vide.

6. Installation (1) selon la revendication 4 ou 5, dont le système de transport (16) est associé par le bas à un tamis sans fin mobile (20) pour supporter le courant de matériau (2) comportant la bande de matériau supérieure (8).

7. Installation (1) selon la revendication 6, dont le tamis sans fin mobile (20) présente, sur sa surface de roulement, une surface de tamis comportant un maillage d'au moins 1 mm, de préférence d'au moins 2 mm et/ou d'au plus 5 mm.

8. Installation (1) selon l'une des revendications 4 à 7, dont le système de transport (16) est associé à un dispositif d'aspiration (24) destiné à aspirer les particules ou fragments libres des noyaux absorbants.
